# EUROPÄISCHE PATENTSCHRIFT

(11) **EP 0 940 131 B1**
(45) Veröffentlichungstag und Bekanntmachung des Hinweises auf die Patenterteilung: **25.10.2006**
(21) Anmeldenummer: 99100986.1
(22) Anmeldetag: 21.01.1999
(51) Int. Cl.: A61F 13/12, A61F 13/02

(54) **Lichtundurchlässiges Augenpflaster**
Light occlusive eye patch
Couvre-oeil étanche à la lumière

(30) Priorität: 06.02.1998 DE 19804665
(43) Veröffentlichungstag der Anmeldung: 08.09.1999
(73) Patentinhaber: Beiersdorf Aktiengesellschaft, 20245 Hamburg (DE)
(72) Erfinder: Lenz, Dirk Dr., 20253 Hamburg (DE); Mayan, Robert Dr., 21614 Buxtehude (DE); Timm, Jürgen, 21218 Seevetal (DE); Trotter, Sebastian, 21244 Buchholz (DE)

(56) Entgegenhaltungen:
- WO-A-89/04649
- US-A- 3 908 645
- US-A- 4 538 603

## Beschreibung

Die Erfindung betrifft einen selbstklebenden Wundschnellverband, der als Okklusionspflaster Verwendung findet.

Okklusionspflaster dienen u.a. dazu, das Schielen insbesondere bei Kindern zu therapieren. Dazu wird das Okklusionspflaster insbesondere über dem funktionstüchtigen Führungsauge des betroffenen Patienten geklebt, um eine Fixation des sehschwachen Auges zu erzwingen.

Bekannt und auf dem Markt erhältlich ist beispielsweise das Okklusionspflaster "Elastopad ®" der Firma Beiersdorf AG, Hamburg. Das Augenpflaster besteht aus einem querelastischen Viskosegewebe-Träger, einem dreischichtigen lichtundurchlässigen Lichtschutzvlies, das auf dem Träger partiell aufgebracht ist, und einer mit Zinkoxid gefüllten Kautschukmasse als Haftklebemasse. Dies Pflaster weist eine relativ gute Luft- und Wasserdampfdurchlässigkeit auf, es ist allerdings nur bei sehr sorgfältiger Plazierung über dem Auge lichtdicht (vollokklusiv). Weitere Auslobungen des Pflasters betreffen die gute Passform, die sichere Haftung und die Anschmiegsamkeit.

Daneben existieren weitere Produkte, die im allgemeinen als Träger ein Gewebe oder Vlies einsetzen, die in Form eines Okklusionspflasters ausgestanzt sind und bei denen zentral ein saugfähiges Pad aufgebracht ist, das mit einer mehr oder weniger lichtdurchlässigen Einlage versehen ist.

Die bekannten Pflaster weisen aber eine Vielzahl von Nachteilen auf.

So ist eine gewünschte Vollokklusivität oft nicht gewährleistet. Bedingt durch die geringere nicht vollflächige Bedeckung des Trägers mit dem Lichtschutzvlies ist ein seitlicher Restlichteinfall insbesondere bei unpräziser Plazierung des Produktes über dem Auge nicht ausgeschlossen. Dieser Effekt kann noch durch Bewegungen des Patienten und damit einhergehender Wanderung des Pflasters verstärkt werden.
Dann bedingt der vielschichtige Aufbau der Okklusionspflaster aus unterschiedlichen Materialien aufwendige und somit relativ teure Herstellungsverfahren.

Aus der DE 40 07 891 A1 ist ein Trägermaterial für medizinische Zwecke aus einem Laminat bekannt, das eine erste polymere Filmschicht, eine zweite, auf der ersten Schicht erzeugte polymere Filmschicht und eine dritte, in der zweiten Schicht zumindest teilweise eingebettet Schicht aus einem makroporösen textilen Material aufweist. Die erste und die zweite Schicht können dabei aus Polyurethan bestehen. Das Aufschäumen der einzelnen Schichten wird aber nicht vorgeschlagen.

WO 89/04649 offenbart ein Augenokklusionspflaster, das aus mehreren Schichten aufgebaut ist und auf der unteren Seite mit einer hautverträglichen selbstklebenden Schicht versehen ist.

US 3908645 offenbart ein Augenokklusionspflaster, das aus mehreren Schichten aufgebaut ist und auf der unteren Seite mit einer selbstklebenden Schicht versehen ist. Die oberste Deckschicht kann dabei geschäumt sein. Zwingend ist wiederum der mehrschichtige Aufbau, wobei das Pad wiederum mehrschichtig aufgebaut ist. Als Schaummaterial für die Deckschicht wird lediglich ein Elastomerschaum, insbesondere Polyvinylchlorid beschrieben.

US 4538603 beschreibt mehrschichtige Wundauflagen, keine Augenokklusionspflaster.

Aufgabe der Erfindung war es, ein Augenokklusionspflaster zur Verfügung zu stellen, das die Nachteile des Standes der Technik vermeidet.

Diese Aufgabe wird durch ein gemäß Hauptanspruch gekennzeichnetes Okklusionspflaster gelöst. Gegenstand der Unteransprüche sind dabei vorteilhafte Weiterbildungen des Pflasters.

Demgemäß betrifft die Erfindung ein Augenokklusionspflaster, das aus einer Schicht aus einem geschäumten Material aus Polyurethan besteht und das auf der unteren Seite mit einer hautverträglichen selbstklebenden Schicht versehen ist. Um eine Okklusivität ausreichend sicherzustellen, ist bei dem einschichtigen Okklusionspflaster eine ausreichende Schichtdicke von mindestens 400 µm zu wählen.

In einer vorteilhaften Ausführungsform besteht das Okklusionspflaster aus einer Deckschicht aus einem geschäumten Material und einer Innenschicht aus einem geschäumten Material, die auf der unteren Seite mit einer hautverträglichen selbstklebenden Schicht versehen ist.

Weiterhin als vorteilhaft hat es sich herausgestellt, wenn auf der Deckschicht eine innenliegende Kontur schwarz bedruckt ist. Diese schwarz bedruckte Kontur stellt bereits bei geringeren Schichtdicken der Deck- beziehungsweise Innenschicht eine Vollokklusivität sicher.

In einer alternativen Ausführungsform ist zwischen Deckschicht und Innenschicht auf der gesamten Fläche eine schwarz eingefärbte Zwischenschicht vorhanden, die ebenfalls aus einem geschäumten Material gebildet wird.

Als geschäumtes Material werden bevorzugt PU-, PP-, PE- oder PVC-Schäume eingesetzt, ganz besonders bevorzugt ist dabei ein PU-Schaum aus Polyesterurethan.

Das geschäumte Material weist weiterhin im trockenen Zustand eine Dichte auf von 0,03 g/cm³ bis 0,8 g/cm³, insbesondere von 0,1 g/cm³ bis 0,3 g/cm³.

Dann hat es sich als vorteilhaft erwiesen, wenn das geschäumte Material eine Schichtdicke aufweist bis 2,0 mm, insbesondere bis 0,8 mm. Insbesondere die kleineren Schichtdicken erreicht man durch Kalandern des geschäumten Materials, was zu einer Kompression desselben führt.

Zur Erhöhung der Schaumdichte, aber auch, um das Einsatzmaterial zu verbilligen, können dem geschäumten Material vorteilhafterweise Füllstoffe wie Calciumcarbonat, Kaolin und/oder Aluminiumerde zugesetzt werden.

Zur Erhöhung der mechanischen Stabilität in Längs- und Querrichtung sowie der Weiterreißfestigkeit der Schäume kann auf der Deckschicht eine Schicht aus nichtgeschäumten PU aufgebracht sein oder in der Deckschicht ein flächiges textiles Gebilde (Vlies, Gewirke, Gewebe) eingebettet sein.

Des weiteren kann auf der klebenden Seite der Innenschicht eine saugfähige Auflage aufgebracht sein, die kleiner als die Klebefläche ist, und zwar bevorzugt in der Mitte des Trägermaterials.

Schließlich beinhaltet eine weitere vorteilhafte Ausführungsform des Pflasters, daß die selbstklebend ausgerüstete Seite mit mindestens einem abziehbaren Deckblatt als Schutzabdeckung versehen ist.

Dann umfaßt der Erfindungsgedanke ein Verfahren zur Herstellung eines Okklusionspflasters, das aus den folgenden Verfahrensschritten besteht.

Der erste Schritt besteht darin, daß die Deckschicht, die Innenschicht sowie gegebenenfalls die Zwischenschicht jeweils in einer Mischung von wäßrigen aliphatischen Dispersionen von Polyesterurethanen vorgelegt werden.

In die jeweiligen Mischungen werden jeweils ein Schäumungsmittel (bevorzugt Ammoniumstearat, Stokal-Produkte, Fa. Stockhausen) sowie ein Farbstoff gegeben.
Neben der schwarz eingefärbten Zwischenschicht werden dabei eine hautfarbene Deckschicht und eine weiße Innenschicht bevorzugt.

Durch das Einblasen von Luft werden die jeweiligen Mischungen auf den gewünschten Schäumungsgrad gebracht.

Der Beschichtungsvorgang beginnt damit, daß Schaum für die Deckschicht auf ein Trennpapier ausgestrichen und anschließend getrocknet wird.
Das Trennpapier oder genauer die besondere Gestaltung der Oberfläche des Trennpapieres führt dazu, daß der getrocknete Schaum beziehungsweise die Deckschicht eine besonders weiche, samtene Anmutung erhalten.

Sofern eine Zwischenschicht vorgesehen ist, wird der Schaum für die Zwischenschicht auf der getrockneten Deckschicht ausgestrichen und anschließend getrocknet.

Der Schaum für die Innenschicht wird analog auf der Deckschicht beziehungsweise der Zwischenschicht ausgestrichen und ebenso getrocknet.

Zur Herstellung der eigentlichen Okklusionspflaster wird zunächst die Klebebeschichtung aufgebracht, anschließend werden die Okklusionspflaster ausgestanzt.

Gegebenenfalls erfolgen noch die Aufbringung einer saugfähigen Auflage mittig auf die Innenschicht sowie eine Einsiegelung der einzelnen Pflaster.

Das erfindungsgemäße Okklusionspflaster weist gegenüber den aus dem Stand der Technik bekannten Pflaster eine Vielzahl von Vorteilen auf.

Die geforderte Vollokklusivität des Okklusionspflasters läßt sich insbesondere bei der vorteilhaften Ausführungsform mit schwarz gefärbter Zwischenschicht in optimaler Weise realisieren. Die gesamte Fläche des Pflasters ist lichtdicht beziehungsweise läßt sich so einstellen.

Deckschicht, Innenschicht und Zwischenschicht können aus einem einzigen Material hergestellt werden, was sehr kostengünstig und wenig aufwendig ist.

Die Anschmiegsamkeit des Pflasters und die Weichheit der Oberfläche sind unübertroffen und für die Anwendung am Auge in idealer Weise geeignet.

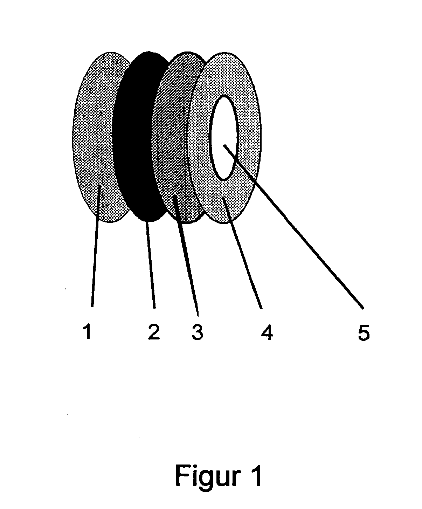

Im folgenden sollen anhand mehrerer Beispiele besonders vorteilhafte Ausführungsformen des Okklusionspflasters dargestellt werden, ohne damit die Erfindung unnötig einschränken zu wollen.
In Beispiel 1 wird gleichzeitig mittels der Figur 1 das erfindungsgemäße Okklusionspflaster näher erläutert.

### Beispiel 1

Das hergestellte Okklusionspflaster setzte sich aus insgesamt drei Lagen PU-Schaum zusammen, und zwar einer hautfarbenen Deckschicht (Auftragsgewicht 77 g/m²) 1, einer schwarz eingefärbten Zwischenschicht 2 (Auftragsgewicht 33 g/m²) und einer weiß eingefärbten Innenschicht 3 (Auftragsgewicht 58 g/m²).

Die innenliegende schwarze Zwischenschicht wurde nach beiden Seiten von den äußeren Schichten gut abgedeckt, dadurch wurde ein Durchscheinen der schwarzen Schicht vermieden.

Die einzelnen Schaumstriche bestanden aus einer Mischung von wäßrigen aliphatischen Dispersionen von Polyesterurethanen (Impranil-Typen, Bayer AG, Leverkusen), die mit einem Schäumungsmittel (Ammoniumstearat, Stokal-Produkte, Fa. Stockhausen) sowie den jeweiligen Farbstoffen (braun, schwarz, weiß) versehen wurden und durch Einblasen von Luft zu einem Schlagschaum einer Dichte von 0,4 g/cm³ verarbeitet wurden.

Auf einer Beschichtungsanlage mit Schuhmesser (Rakel) und einem Gebläse- oder Strahlentrockner wurde der erste Schlagschaum bei einem Rakelspalt zwischen 0,5 bis 0,7 mm auf ein Trennpapier (Warren Stripkote) aufgetragen und abgerakelt. Die Trocknung des Schaumes erfolgte im Trockenkanal bei ansteigender Temperatur von 70 °C auf 150 °C in mehreren Zonen auf einer Länge von 15 m.

Der Auftrag der zweiten Schicht Schlagschaum mit dem anderen Farbton erfolgte direkt auf den zuvor getrockneten ersten PU-Schaum. Auf die zweite Schicht wurde anschließend die dritte Schicht aufgetragen.

Daraus resultierte ein fester Verbund der drei Schaumschichten, die zusammen eine Dicke von 0,8 mm und ein Flächengewicht von 170 g/m² besaßen und absolute Lichtundurchlässigkeit im Bereich des sichtbaren Lichts (400 bis 700 nm) zeigten. Darüber hinaus wies das Material eine Luftdurchlässigkeit und eine extrem gute Wasserdampfdurchlässigkeit auf.

Einzelne Werte können der Tabelle 2 entnommen werden.

Auf die Innenschicht 3 wurde vollflächig eine Klebebeschichtung 4 aufgetragen, auf die wiederum mittig eine saugfähige Auflage 5 aus einem Vlies aufgebracht war. Die Kontur des Pflasters war dabei der Kontur der Augenhöhle entsprechend angepaßt.

### Beispiel 2

In analoger Weise zu Beispiel 1 wurde ein mehrlagiges Okklusionspflaster durch Beschichten mit einem PU-Schlagschaum gleicher Dichte auf Basis aliphatischer Polyesterurethane (Impranil-Typen, Bayer AG, Leverkusen) hergestellt.

Als weitere Schicht auf die Deckschicht wurde ein kompakter Deckstrich (Impraperm-Type, Bayer AG, Leverkusen) aufgetragen, also kein Schaum, und zwar wurde dieser mit einem Flächengewicht von 11 g/m² aufgebracht.

Diese Schicht sollte die mechanische Stabilität in Längs- und Querrichtung sowie die Weiterreißfestigkeit der Schäume erhöhen.

Die schwarze Zwischenschicht hatte in diesem Ausführungsbeispiel ein Flächengewicht von 55 g/m².
Die veränderte Ausführung zeigte deutlich höhere Höchstzugkräfte in Längs- und Querrichtung. Man erreichte eine (immer noch) extrem gute Wasserdampfdurchlässigkeit, aber keine Luftdurchlässigkeit mehr.

### Beispiel 3

Als Ausgangsmaterial wurde ein einlagiger Treibschaum auf Basis aromatischer ZweiKomponenten Polyurethane (High-Solids-Typen, Fa. Bayer, Leverkusen) bei einem Flächengewicht von 250 g/m² und einer Dicke von 0,4 mm gewählt.
Das Material zeigte eine im Vergleich zu den Schlagschäumen auf Basis von wäßrigen aliphatischen Polyesterurethanen gemäß den Beispielen 1 und 2 eine deutlich höhere Reißfestigkeit in Längs- und Querrichtung sowie eine deutlich höhere Dichte, des weiteren ebenfalls eine extrem gute Wasserdampfdurchlässigkeit.

Den im folgenden abgebildeten Tabellen kann in übersichtlicher Art und Weise nochmals der Aufbau der erfindungsgemäß ausgestalteten Okklusionspflastem gemäß den vorhergehenden Beispielen entnommen werden. Die zweite Tabelle gibt Aufschluß über die physikalische Eigenschaften der Okklusionspflaster.

**Tabelle 1: Aufbau der Okklusionspflaster gemäß den Beispielen 1 bis 3**

| **Aufbau** | **Beispiel 1** | **Beispiel 2** | **Beispiel 3** |
|---|---|---|---|
| Deckstrich hautfarben | ― | 11 g/qm | ― |
| Deckschicht hautfarben | 77 g/m² | 108 g/m² | 250 g/m² |
| Zwischenschicht schwarz | 33 g/m² | 55 g/m² | ― |
| Innenschicht weiß | 58 g/m² | 74 g/m² | ― |

**Tabelle 2: Physikalische Eigenschaften der Okklusionspflaster gemäß den Beispielen 1 bis 3**

| Methode | **Beispiel 1** | **Beispiel 2** | **Beispiel 3** |
|---|---|---|---|
| Flächengewicht | 170 g/m² | 250 g/m² | 250 g/m² |
| Dicke | 0,8 mm | 1,1 mm | 0,4 mm |
| Dichte (getrockneter Zustand) | 0,212 g/cm³ | 0,220 g/cm³ | 0,625 g/cm³ |
| Höchstzugkraft längs (DIN EN ISO 527) | 6,8 N/cm | 9 N/cm | 11 N/cm |
| Höchstzugkraft-Dehnung längs (DIN EN ISO 527) | 400 % | 410% | 400% |
| Höchstzugkraft quer (DIN EN ISO 527) | 6,3 N/cm | 12,5 N/cm | 11 N/cm |
| Höchstzugkraft-Dehnung quer (DIN EN ISO 527) | **470%** | **540%** | **400 %** |
| Luftdurchlässigkeit Gurley-Densometer (Gurley, Troy Instruments, New York, USA) | 40 cm³/(cm²s) | ― | ― |
| Wasserdampfdurchlässigkeit Methode angelehnt an DAB 10*) | 5500 g/(m²*24h) | 2900 g/(m²*24h) | 1900 g/(m²*24h) |

| | | | |
|---|---|---|---|
| *) Eine Materialprobe wird dicht über einen Probenkörper (nach DAB 10 spezifiziert) befestigt, der mit 15 ml Wasser gefüllt ist. Das Probengefäß wird im Klimaschrank bei 37 °C, 30 % rel. Luftfeuchte für 24 h belassen und durch Rückwägung die Wasserdampfdurchlässigkeit in g/(m²24 h) bestimmt. | | | |

## Patentansprüche

1. Augenakklusionspflaster, bestehend aus einer Schicht aus einem geschäumten Material, das auf der unteren Seite mit einer hautverträglichen selbstklebenden Schicht versehen ist, **dadurch gekennzeichnet, dass** das geschäumte Material Polyurethan ist und im trockenen Zustand eine Dichte von 0,03 g/cm³ bis 0,8 g/cm³ und eine Schichtdicke von mindestens 400 µm aufweist.

2. Okklusionspflaster nach Anspruch 1, bestehend aus einer Deckschicht aus geschäumten Polyurethan und einer Innenschicht aus einem geschäumten Polyurethan, die auf der unteren Seite mit einer hautverträglichen selbstklebenden Schicht versehen ist.

3. Okklusionspflaster nach Anspruch 2, **dadurch gekennzeichnet, dass** auf der Deckschicht eine innenliegende Kontur schwarz bedruckt ist.

4. Okklusionspflaster nach Anspruch 2, **dadurch gekennzeichnet, dass** zwischen Deckschicht und Innenschicht auf der gesamten Fläche eine schwarz eingefärbte Zwischenschicht aus einem geschäumten Material vorhanden ist.

5. Okklusionspflaster nach Anspruch 4, **dadurch gekennzeichnet, dass** als geschäumtes Material für die Zwischenschicht PU-, PP-, PE- oder PVC-Schäume eingesetzt werden.

6. Okklusionspflaster nach einem der Ansprüche 1 bis 5, **dadurch gekennzeichnet, dass** als PU-Schaum ein Polyesterurethan eingesetzt wird.

7. Okklusionspflaster nach einem der Ansprüche 1 bis 6, **dadurch gekennzeichnet, dass** das geschäumte Material im trockenen Zustand eine Dichte aufweist von 0.1 g/cm³ bis 0,3 g/cm³.

8. Okklusionspflaster nach einem der Ansprüche 1 bis 7, **dadurch gekennzeichnet, dass** das geschäumte Material eine Schichtdicke bis 2,0 mm, insbesondere bis 0,8 mm, aufweist.

9. Okktusionspftsster nach einem der Ansprüche 1 bis 8, **dadurch gekennzeichnet, dass** dem geschäumten Material Füllstoffe wie Calciumcarbonat, Kaolin und/oder Aluminiumerde zugesetzt sind.

10. Okklusionspflaster nach einem der Ansprüche 1 bis 9, **dadurch gekennzeichnet, dass** auf der Deckschicht eine Schicht aus nichtgeschäumten PU aufgebracht ist oder in der Deckschicht ein flächiges textiles Gebilde (Vlies, Gewirke, Gewebe) eingebettet ist.

11. Okklusionspflaster nach einem der Ansprüche 1 bis 10, **dadurch gekennzeichnet, dass** auf der klebenden Seite der Innenschicht eine Wundauflage aufgebracht ist, die kleiner als die Klebefläche ist.

12. Okklusionspflaster nach einem der Ansprüche 1 bis 11, **dadurch gekennzeichnet, dass** die selbstklebend ausgerüstete Seite mit mindestens einem abziehbaren Deckblatt als Schutzabdeckung versehen ist.

## Claims

1. Occlusive eye patch, consisting of a layer of a foamed material which is provided on the lower side with a skin-compatible self-adhesive layer, **characterized in that** the foamed material is polyurethane and, in the dry state, has a density of 0.03 g/cm³ to 0.8 g/cm³ and a layer thickness of at least 400 µm.

2. Occlusive patch according to Claim 1, consisting of a top layer made of foamed polyurethane and an inner layer made of a foamed polyurethane, which inner layer is provided on the lower side with a skin-compatible self-adhesive layer.

3. Occlusive patch according to Claim 2, **characterized in that** an internal contour is printed in black on the top layer.

4. Occlusive patch according to Claim 2, **characterized in that** a black-coloured intermediate layer made of a foamed material is present between top layer and inner layer across the entire surface area.

5. Occlusive patch according to Claim 4, **characterized in that** PU, PP, PE or PVC foams are used as foamed material for the intermediate layer.

6. Occlusive patch according to one of Claims 1 to 5, **characterized in that** a polyester urethane is used **as PU foam.**

7. Occlusive patch according to one of Claims 1 to 6, **characterized in that** the foamed material in the dry state has a density of 0.1 g/cm³ to 0.3 g/cm³.

8. Occlusive patch according to one of Claims 1 to 7, **characterized in that** the foamed material has a layer thickness of up to 2.0 mm, in particular of up to 0.8 mm.

9. Occlusive patch according to one of Claims 1 to 8, **characterized in that** fillers such as calcium carbonate, kaolin and/or alumina are added to the foamed material.

10. Occlusive patch according to one of Claims 1 to 9, **characterized in that** a layer of unfoamed PU is applied to the top layer, or a planar textile structure (nonwoven, knit, woven) is embedded in the top layer.

11. Occlusive patch according to one of Claims 1 to 10, **characterized in that** a wound pad smaller than the adhesion area is applied to the adhesive side of the inner layer.

12. Occlusive patch according to one of Claims 1 to 11, **characterized in that** the self-adhesive side is provided with at least one peelable cover sheet as a protective covering.

## Revendications

1. Pansement d'occlusion oculaire, se composant d'une couche faite d'un matériau alvéolaire, qui est pourvu, sur la face inférieure, d'une couche autoadhésive compatible avec la peau, **caractérisé en ce que** le matériau alvéolaire est le polyuréthanne et **en ce qu'**il présente, à l'état sec, une densité de 0,03 g/cm³ à 0,8 g/cm³ et une épaisseur de couche d'au moins 400 µm.

2. Pansement d'occlusion selon la revendication 1, se composant d'une couche de finition en polyuréthanne alvéolaire et d'une couche interne en polyuréthanne alvéolaire, qui est pourvue, sur la face inférieure d'une couche autoadhésive compatible avec la peau.

3. Pansement d'occlusion selon la revendication 2, **caractérisé en ce qu'**un contour situé à l'intérieur est imprimé en noir sur la couche de finition.

4. Pansement d'occlusion selon la revendication 2, **caractérisé en ce qu'**entre la couche de finition et la couche interne est présente, sur la totalité de la surface, une couche intermédiaire colorée en noir faite d'un matériau alvéolaire.

5. Pansement d'occlusion selon la revendication 4, **caractérisé en ce que** l'on utilise, en tant que matériau alvéolaire pour la couche intermédiaire, des mousses de PU, de PP, de PE ou de PVC.

6. Pansement d'occlusion selon l'une quelconque des revendications 1 à 5, **caractérisé en ce que** l'on utilise, en tant que mousse de PU, un polyesteruréthanne.

7. Pansement d'occlusion selon l'une quelconque des revendications 1 à 6, **caractérisé en ce que** le matériau alvéolaire présente, à l'état sec, une densité d'au moins 0,1 g/cm³ à 0,3 g/cm³.

8. Pansement d'occlusion selon l'une quelconque des revendications 1 à 7, **caractérisé en ce que** le matériau alvéolaire présente, à l'état sec, une épaisseur de couche allant jusqu'à 2,0 mm, en particulier jusqu'à 0,8 mm.

9. Pansement d'occlusion selon l'une quelconque des revendications 1 à 8, **caractérisé en ce que** l'on ajoute au matériau alvéolaire des charges comme le carbonate de calcium, le kaolin et/ou des terres d'aluminium.

10. Pansement d'occlusion selon l'une quelconque des revendications 1 à 9, **caractérisé en ce que**, sur la couche de finition, est appliquée une couche en PU non alvéolaire ou **en ce que**, dans la couche de finition, est noyée une structure textile plane (non tissé, tissu à mailles, tissu).

11. Pansement d'occlusion selon l'une quelconque des revendications 1 à 10, **caractérisé en ce que**, sur la face collante de la couche interne, est appliqué un pansement, qui est plus petit que la surface de collage.

12. Pansement d'occlusion selon l'une quelconque des revendications 1 à 11, **caractérisé en ce que** la face équipée d'une manière auto-adhésive est pourvue d'au moins une feuille de recouvrement en tant que couverture de protection.
